(19) Europäisches Patentamt
European Patent Office
Office européen des brevets

(11) Veröffentlichungsnummer: **0 001 108**
**B1**

(12) # EUROPÄISCHE PATENTSCHRIFT

(45) Veröffentlichungstag der Patentschrift:
**29.04.81**

(21) Anmeldenummer: **78100826.3**

(22) Anmeldetag: **06.09.78**

(51) Int. Cl.³: **C 07 D 237/04,** C 07 D 405/04,
C 07 D 405/12, C 07 D 409/04,
A 61 K 31/50

(54) **Neue 1,4-Dihydropyridazine, Verfahren zu ihrer Herstellung sowie ihre Verwendung in Arzneimitteln.**

(30) Priorität: **14.09.77 DE 2741260**

(43) Veröffentlichungstag der Anmeldung:
**21.03.79 Patentblatt 79/6**

(45) Bekanntmachung des Hinweises auf die Patenterteilung:
**29.04.81 Patentblatt 81/17**

(84) Benannte Vertragsstaaten:
**BE CH DE FR GB NL**

(56) Entgegenhaltungen:
**US-A-3 022 305**

(73) Patentinhaber: **BAYER AG, Zentralbereich Patente, Marken und Lizenzen, D-5090 Leverkusen 1, Bayerwerk (DE)**

(72) Erfinder: **Meyer, Horst, Dr., Theodor-Heuss-Strasse 110, D-5600 Wuppertal 1 (DE)**
Erfinder: **Franckowiak, Gerhard, Dr., Pahlkestrasse 17, D-5600 Wuppertal 1 (DE)**
Erfinder: **Bossert, Friedrich, Dr., Claudiusweg 7, D-5600 Wuppertal 1 (DE)**
Erfinder: **Heise, Arend, Dr., Moebeck 50, D-5600 Wuppertal 1 (DE)**
Erfinder: **Kazda, Stanislav, Dr., Pahlkestrasse 55N, D-5600 Wuppertal 1 (DE)**
Erfinder: **Stoepel, Kurt, Dr., In den Birken 69, D-5600 Wuppertal 1 (DE)**
Erfinder: **Towart, Robertson, Dr., Claudiusweg 9, D-5600 Wuppertal 1 (DE)**
Erfinder: **Wehinger, Egbert, Dr., Donnenbergerstrasse 90, D-5620 Velbert 15 (DE)**

EP 0 001 108 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

BUNDESDRUCKEREI BERLIN

Neue 1,4-Dihydropyridazine, Verfahren zu ihrer Herstellung sowie ihre Verwendung in Arzneimitteln

Die vorliegende Erfindung betrifft neue 1,4-Dihydropyridazine, ein Verfahren zu ihrer Herstellung sowie ihre Verwendung in Arzneimitteln, insbesondere in kreislaufbeeinflussenden und spasmolytischen Mitteln.

Es ist bereits bekanntgeworden, daß man 1,4-Dihydropyridazine erhält, wenn man substituierte 1,4-Dicarbonylverbindungen mit Hydrazinhydrat umsetzt (vgl. W. Borsche und M. Spannagel, Liebigs Ann. Chem., 331, 300 ff [1904]).

Aus dem US-Patent 3 022 305 sind fluorhaltige Pyridazine und ein spezielles Verfahren zu ihrer Herstellung bekannt. Nach dem dort beschriebenen Verfahren lassen sich nur solche Pyridazine herstellen, die in 3- und 6-Position jeweils identische Fluoralkylsubstituenten tragen. Sie unterscheiden sich damit chemisch eindeutig von den erfindungsgemäßen Verbindungen. Sie sollen als Antioxidantien verwendet werden können. Eine therapeutische Wirkung ist nicht beschrieben.

Die vorliegende Erfindung betrifft neue 1,4-Dihydropyridazine der allgemeinen Formel (I)

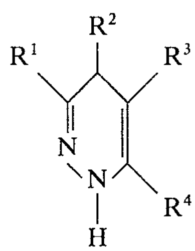

(I)

in welcher

$R^1$ für Wasserstoff, geradkettiges, verzweigtes oder cyclisches Alkyl oder Alkenyl mit bis zu 8 Kohlenstoffatomen steht, wobei die Alkylkette gegebenenfalls durch ein Sauerstoffatom unterbrochen ist oder wobei die Alkylkette substituiert ist durch Halogen, Phenoxy, Halogenphenyl, Nitrophenyl, Pyridyl, Furyl, Thienyl, Dialkyl- oder Benzylalkylamin mit je 1 bis 2 Kohlenstoffatomen im Alkylrest, oder für einen Pyridyl-, Furyl-, Thienyl-, Pyrryl- oder Phenylrest steht, wobei der Phenylrest gegebenenfalls durch 1 oder 2 gleiche oder verschiedene Substituenten aus der Gruppe Halogen, Nitro, Cyano, Trifluormethyl, Trifluormethoxy, Alkyl oder Alkoxy mit jeweils 1 bis 4 Kohlenstoffatomen substituiert ist,

$R^2$ für einen Phenylrest steht, der gegebenenfalls durch 1 oder 2 gleiche oder verschiedene Substituenten aus der Gruppe Halogen, Nitro, Cyano, Trifluormethyl, Trifluormethoxy, Alkyl, Alkenyl, Alkoxy oder Alkylmercapto mit jeweils 1 bis 4 Kohlenstoffatomen substituiert ist, oder für einen Naphthyl-, Pyridyl-, Thienyl-, Pyrryl- oder Furylrest steht,

$R^3$ für Wasserstoff oder die Gruppe $COR^5$ steht, wobei $R^5$ für einen Hydrazinorest oder einen Alkylrest mit 1 bis 4 Kohlenstoffatomen steht, oder $R^5$ und $R^4$ gemeinsam einen 5- bis 6gliedrigen Ring bilden, der gegebenenfalls noch ein Sauerstoffatom als Heteroatom enthält, oder wobei $R^5$ für die Gruppe $OR^6$, wobei $R^6$ für geradkettiges, verzweigtes oder cyclisches Alkyl, Alkenyl oder Alkinyl mit bis zu 6 Kohlenstoffatomen steht, wobei die Alkyl- und Alkenylgruppe gegebenenfalls durch ein Sauerstoffatom unterbrochen ist, oder wobei die Alkylkette gegebenenfalls substituiert ist durch Phenyl, Phenoxy, Halogenphenyl, Nitrophenyl, Pyridyl, Dialkylamin oder Benzylalkylamin mit jeweils 1 bis 2 Kohlenstoffatomen im Alkylrest, und

$R^4$ für einen Alkylrest mit 1 bis 4 Kohlenstoffatomen steht, der mit $R^5$ einen 5- bis 6gliedrigen Ring bilden kann, der gegebenenfalls als Heteroatom ein Sauerstoff enthält oder für einen Phenyl-, Benzyl-, Pyridyl- oder Thienylrest steht.

Es wurde gefunden, daß man die neuen 1,4-Dihydropyridazine der Formel (I) erhält, wenn man 1,4-Dicarbonylverbindungen der Formel (II)

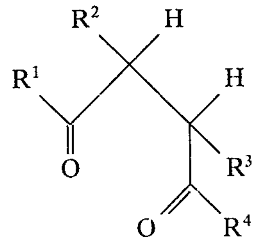

(II)

in welcher

$R^1$, $R^2$, $R^3$ und $R^4$ die oben angegebene Bedeutung haben, mit Hydrazin der Formel (III)

$$H_2N—NH_2 \qquad (III)$$

in organischen Lösungsmitteln umsetzt.

Die neuen erfindungsgemäßen 1,4-Dihydropyridazine besitzen wertvolle pharmakologische Eigenschaften. Aufgrund ihrer kreislaufbeeinflussenden und spasmolytischen Wirkungen können sie als antihypertensive Mittel, als Vasodilatatoren sowie als Coronartherapeutika und Spasmolytika Verwendung finden. Von den bisher bekannten Dihydropyridazinen sind keine therapeutischen Wirkungen, insbesondere keine Wirkungen auf den Kreislauf bekanntgeworden. Die erfindungsgemäßen Verbindungen stellen somit eine neuartige Stoffklasse zur Kreislaufbehandlung, sowie zur Behandlung bei Erkrankungen des Magen-Darm-Traktes, des Urogenital-Traktes und des Respirations-Systems dar und sind somit als eine Bereicherung der Pharmazie anzusehen.

Als Beispiel für die unerwartet vorteilhafte Kreislaufwirkung sei die Erhöhung der zerebralen Durchblutung angeführt. Hierzu wird radioaktives Xenon in das Gehirn von Katzen gegeben und dann die Erhöhung der zerebralen Durchblutung als Funktion der Ausspülgeschwindigkeit der Radioaktivität gemessen. Es zeigt sich, daß nach intravenöser Applikation der erfindungsgemäßen Verbindungen in der in der Tabelle angegebenen Grenzdosis der zerebrale Durchfluß signifikant, d. h. um mindestens 15% gesteigert wird.

Tabelle

| Verbindung Beispiel-Nr. | Signifikanter Anstieg (≥ 15%) der cerebralen Durchblutung (Xenon-Clearence; Katze) Grenzdosis mg/kg i. v. |
|---|---|
| 7 | 0,1 |
| 10 | 0,1 |
| 15 | 0,05 |
| 17 | 0,001 |
| 18 | 0,05 |
| 22 | 0,01 |
| 24 | 0,001 |
| 27 | 0,01 |
| 28 | 0,001 |
| 29 | 0,001 |
| 29 | 0,01 |
| 30 | 0,001 |
| 31 | 0,001 |
| 34 | 0,001 |
| 35 | 0,01 |
| 37 | 0,01 |
| 38 | 0,01 |
| 41 | 0,1 |

Verwendet man 2-Acetyl-3-(2′-nitrophenyl)-4-oxo-hexancarbonsäureethylester und Hydrazin als Ausgangsstoffe, so kann der Reaktionsablauf durch das folgende Formelschema beispielhaft wiedergegeben werden:

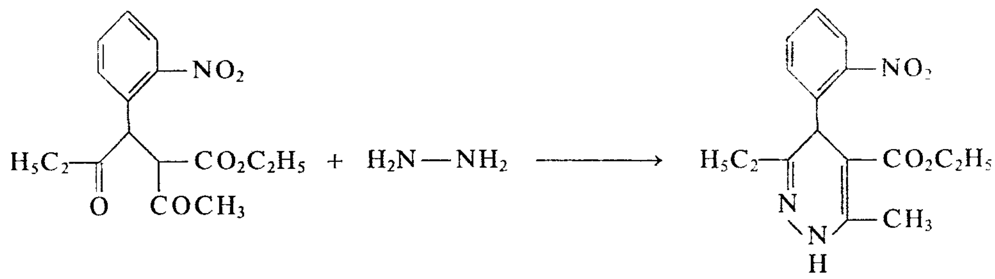

Gemäß der angegebenen Verfahrensweise wird eine 1,4-Dicarbonylverbindung der Formel II mit Hydrazin der Formel (III) zu einem 1,4-Dihydropyridazinderivat der Formel (I) umgesetzt.

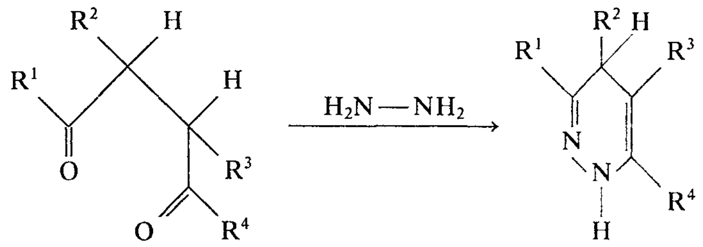


(II) (I)

Die als Ausgangsstoffe verwendeten 1,4-Dicarbonylverbindungen der Formel (II) sind teils literaturbekannt oder können nach literaturbekannten Methoden hergestellt werden (vgl. H. Stetter, Angew. Chem., 88, 695 ff [1976], und F. Boberg und A. Kieso, Liebigs Ann. Chem., 626, 71 ff [1959]).

Die genannten 1,4-Dicarbonylverbindungen der Formel (II) können als solche oder in Form entsprechender 1-Oxo-4-nitroderivate eingesetzt werden. Aus den Nitroverbindungen kann mit Hilfe der Nef-Reaktion das entsprechende 1,4-Diketon in situ hergestellt werden (vgl. F. Boberg und G. R. Schultze, Chem. Ber., 90, 1215 ff [1957]).

Das als Ausgangsstoff verwendete Hydrazin ist literaturbekannt (vgl. L. F. Andrieth und B. A. Ogg. The Chemistry of Hydrazine, Hohn Wiley u. Sons Inc., New York, N. Y., 1951).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise bei 40 bis 120°C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird 1 Mol der 1,4-Dicarbonylverbindung der Formel (II) mit einem Mol der Hydrazinverbindung der Formel (III) in einem geeigneten Lösungsmittel zur Reaktion gebracht.

Die Isolierung und Reinigung der erfindungsgemäßen Substanzen erfolgt vorzugsweise derart, daß man gegebenenfalls nach Abtrennung unlöslicher Stoffe das Lösungsmittel im Vakuum abdestilliert und den gegebenenfalls erst nach Eiskühlung kristallin erhaltenen Rückstand aus einem geeigneten Lösungsmittel umkristallisiert.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomer) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E. L. Eliel, Stereochemistry of Carbon Compounds, McGraw-Hill, 1962).

Außer den unten aufgeführten Herstellungsbeispielen seien folgende erfindungsgemäße Wirkstoffe genannt:

1,4-Dihydro-3,6-dimethyl-4-(3'-nitrophenyl)-pyridazin-5-carbonsäureäthylester,
3-Äthyl-1,4-dihydro-6-methyl-4-(2'-nitrophenyl)-pyridazin-5-carbonsäureisopropylester,
4-(2'-Cyanophenyl)-1,4-dihydro-3,6-dimethyl-pyridazin-5-carbonsäureäthylester,
6-Äthyl-1,4-dihydro-3-methyl-4-(4'-trifluormethylphenyl)-pyridazin-5-carbonsäuremethylester,
3-Äthyl-1,4-dihydro-4-(4'-methoxyphenyl)-6-methyl-pyridazin-5-carbonsäureäthylester,
1,4-Dihydro-3,6-dimethyl-4-(2'-nitrophenyl)-pyridazin-5-carbonsäure-(β-methoxyäthyl)-ester,
3-Äthyl-1,4-dihydro-6-methyl-4-(pyridyl-(2))-pyridazin-5-carbonsäureäthylester,
3-n-Butyl-4-(3'-chlorphenyl)-1,4-dihydro-6-methyl-pyridazin-5-carbonsäure-t-butylester,
3-Äthyl-1,4-dihydro-6-methyl-4-(4'-trifluormethylmercapto)-pyridazin-5-carbonsäuremethylester,
1,4-Dihydro-3,6-dimethyl-4-(2'-nitrophenyl)-pyridazin-5-carbonsäureisopropylester,
1,4-Dihydro-3,6-dimethyl-4-(3'-nitrophenyl)-pyridazin-5-carbonsäureisopropylester,
3,6-Diäthyl-1,4-dihydro-4-(2'-nitrophenyl)-pyridazin-5-carbonsäure-n-propylester,
3-Cyclohexyl-1,4-dihydro-6-methyl-4-(4'-trifluormethylphenyl)-pyridazin-5-carbonsäure-t-butylester,
4-(3'-Chlorphenyl)-1,4-dihydro-3-n-hexyl-6-methyl-pyridazin-5-carbonsäureäthylester,
3-Äthyl-1,4-dihydro-6-methyl-4-(2'-nitrophenyl)-pyridazin-5-carbonsäure-t-butylester,
3-Äthyl-1,4-dihydro-6-methyl-4-(2'-nitrophenyl)-pyridazin-5-carbonsäure-(β-methoxyäthyl)-ester,
3-Äthyl-1,4-dihydro-6-methyl-4-(3'-nitrophenyl)-pyridazin-5-carbonsäure-(β-phenoxyäthyl)-ester,
3-(β-n-Butoxyäthyl)-1,4-dihydro-6-methyl-4-(3'-nitrophenyl)-pyridazin-5-carbonsäureäthylester,
3-Äthyl-1,4-dihydro-6-methyl-4-(3'-nitrophenyl)-pyridazin-5-carbonsäure-(β,β'-hexafluoro-isopropyl)-ester,
3-Äthyl-1,4-dihydro-4-(thienyl-(2))-6-trifluormethyl-pyridazin-5-carbonsäureäthylester,
3-(β-Äthoxyäthyl)-4-(3'-chlorphenyl)-1,4-dihydro-6-methyl-pyridazin-5-carbonsäuremethylester,
3-Äthyl-1,4-dihydro-6-methyl-4-(pyridyl-(2))-pyridazin-5-carbonsäure-(β-dimethylaminoäthyl)-ester,
3,6-Diäthyl-1,4-dihydro-4-(2'-trifluormethylphenyl)-pyridazin-5-carbonsäureäthylester,
1,4-Dihydro-6-methyl-4-(3'-nitrophenyl)-pyridazin-5-carbonsäureäthylester,
3-Äthyl-1,4-dihydro-4-(3'-nitrophenyl)-6-phenyl-pyridazin-5-carbonsäuremethylester,
3-Äthyl-1,4,5,6,7,8-hexahydro-5-oxo-4-(3'-nitrophenyl)-cinnolin,
1,4-Dihydro-6-methyl-4-(3'-nitrophenyl)-3-pentafluoräthyl-pyridazin-5-carbonsäureäthylester,
3-(β-(3'-Chlorphenyl)-äthyl)-1,4-dihydro-6-methyl-4-(3'-nitrophenyl)-pyridazin-5-carbonsäuremethylester,
3-Äthyl-4-(2'-cyanophenyl)-1,4-dihydro-6-methyl-pyridazin-5-carbonsäure-(β-äthylmercapto-äthyl)-ester,
1,4-Dihydro-3-(furyl-(2))-6-methyl-4-(2'-trifluormethylphenyl)-pyridazin-5-carbonsäure-n-propylester,
3-Äthyl-1,4-dihydro-4,6-di-(pyridyl-(3))-pyridazin,
3-(β-Äthylmercaptoäthyl)-1,4-dihydro-6-methyl-4-(2'-trifluormethylphenyl)-pyridazin-5-carbonsäure-n-propylester,
3-Äthyl-4-(3'-nitrophenyl)-5-oxo-1,4,5,7-tetrahydrofuro[4,3-c]pyridazin,
3-Äthyl-6-acetyloxymethyl-1,4-dihydro-4-(3'-nitrophenyl)-pyridazin-5-carbonsäureäthylester,
1,4-Dihydro-3,6-dimethyl-4-(isochinolyl-(1))-pyridazin-5-carbonsäure-(β-methoxyäthyl)-ester,
3-Äthyl-1,4-dihydro-6-methyl-4-(3'-methylsulfonylphenyl)-pyridazin-5-carbonsäureäthylester,
5-Acetyl-3-äthyl-4-(3'-chlorphenyl)-1,4-dihydro-6-methyl-pyridazin,
3-Äthyl-5-benzoyl-4-(2'-cyanophenyl)-1,4-dihydro-6-methyl-pyridazin,
5-Acetyl-3-n-butyl-1,4-dihydro-4-(3'-methylmercaptophenyl)-pyridazin.

Die neuen Verbindungen haben ein breites und vielseitiges pharmakologisches Wirkungsspektrum. Im einzelnen konnten im Tierexperiment folgende Hauptwirkungen nachgewiesen werden:

1) Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.
2) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3) Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren.
4) Die Verbindungen senken den Blutdruck von normotonen und hypertonen Tieren und können somit als antihypertensive Mittel verwendet werden.

5) Die Verbindungen haben stark muskulär-spasmolytische Wirkungen, die an der glatten Muskulator des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Die Erfindung betrifft auch solche Formulierungen, die neben Verbindungen der Formel (I) auch andere Wirkstoffe enthalten, insbesondere solche Wirkstoffe, die als Kombinationspartner von kreislaufbeeinflussenden und spasmolytisch wirkenden Substanzen gebräuchlich sind, wie z. B. Saluretica, Diuretica, β-Blocker.

Die Applikation erfolgt in üblicher Weise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten.

Weiterhin können Gleitmittel, wie Magnesiumstearat, Natriumlaurylsulfat und Talkum zum Tablettieren mitverwendet werden Im Falle wäßriger Suspension und/oder Elixieren, die für orale Anwendung gedacht sind, können die Wirkstoffe außer mit den obengenannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,0001 bis 10 mg/kg, vorzugsweise etwa 0,005 bis 5 mg/kg Körpergewicht pro Tag zur Erzielung wirksamer Ergebnisse zu verabreichen, und bei oraler Applikation beträgt die Dosierung etwa 0,01 bis 20 mg/kg, vorzugsweise 0,1 bis 5 mg/kg Körpergewicht pro Tag.

## Herstellungsbeispiele

### Beispiel 1

1,4-Dihydro-3,6-dimethyl-4-phenyl-pyridazin-5-carbonsäuremethylester

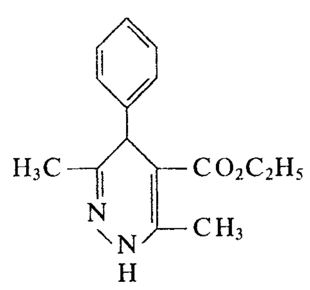

19 g (75 mMol) 2-Acetyl-4-oxo-3-phenyl-pentancarbonsäureethylester wurden zusammen mit 3,75 g (75 mMol) Hydrazinhydrat in 100 ml Ethanol 10 Stunden unter Stickstoff zum Sieden erhitzt. Anschließend wurde das Lösungsmittel im Vakuum abdestilliert und der Rückstand aus Isopropanol umkristallisiert. Schmp. 118° C, Ausbeute 48% der Theorie.

Beispiel 2

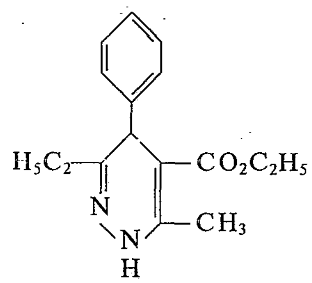

Analog Beispiel 1 wurde durch Umsetzung von 2-Acetyl-4-oxo-3-phenyl-hexancarbonsäureethylester mit Hydrazin 3-Ethyl-1,4-dihydro-6-methyl-4-phenyl-pyridazin-5-carbonsäureethylester vom Schmp. 109° C (Isopropanol) erhalten. Ausbeute 46% der Theorie.

Beispiel 3

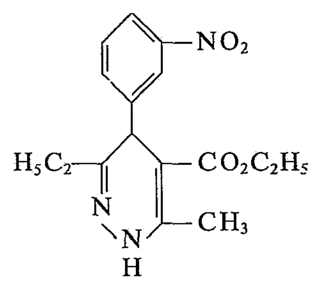

Analog Beispiel 1 wurde durch Umsetzung von 2-Acetyl-4-oxo-3-(3′-nitrophenyl)-hexancarbonsäureethylester mit Hydrazin 3-Ethyl-1,4-dihydro-6-methyl-4-(3′-nitrophenyl)-pyridazin-5-carbonsäureethylester vom Schmp. 124° C (Isopropanol) erhalten. Ausbeute 21,7% der Theorie.

Beispiel 4

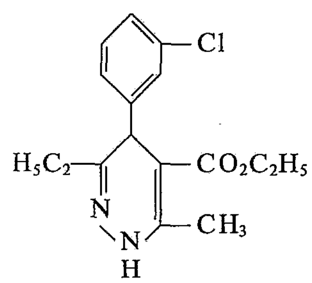

Analog Beispiel 1 wurde durch Umsetzung von 2-Acetyl-3-(3′-chlorphinyl)-4-oxo-hexancarbonsäureethylester mit Hydrazin 3-Ethyl-4-(3′-chlorphenyl)-1,4-dihydro-6-methyl-pyridazin-5-carbonsäureethylester vom Schmp. 112° C (Isopropanol) erhalten. Ausbeute 50,2% der Theorie.

Beispiel 5

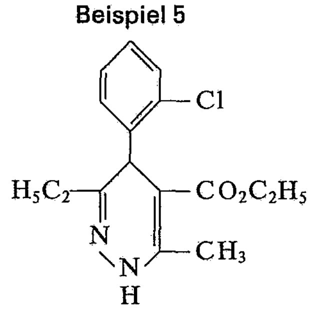

Analog Beispiel 1 wurde durch Umsetzung von 2-Acetyl-3-(2′-chlorphenyl)-4-oxo-hexancarbonsäureethylester mit Hydrazin 3-Ethyl-4-(2′-chlorphenyl)-1,4-dihydro-6-methyl-pyridazin-5-carbonsäureethylester vom Schmp. 112° C (Isopropanol) erhalten. Ausbeute 48% der Theorie.

Beispiel 6

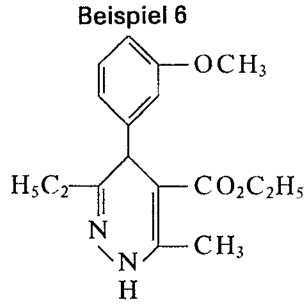

Analog Beispiel 1 wurde durch Umsetzung von 2-Acetyl-3-(3'-methoxyphenyl)-4-oxo-hexancarbonsäureethylester mit Hydrazin 3-Ethyl-1,4-dihydro-4-(3'-methoxyphenyl)-6-methyl-pyridazin-5-carbonsäureethylester vom Schmp. 89° C (Isopropanol) erhalten. Ausbeute 56% der Theorie.

Beispiel 7

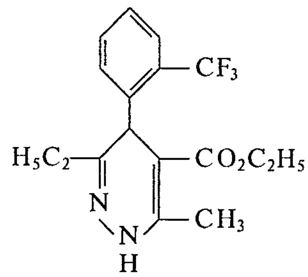

Analog Beispiel 1 wurde durch Umsetzung von 2-Acetyl-4-oxo-3-(2'-trifluormethylphenyl)-hexancarbonsäureethylester mit Hydrazin 3-Ethyl-1,4-dihydro-6-methyl-4-(2'-trifluormethylphenyl)-pyridazin-5-carbonsäureethylester vom Schmp. 124° C (Isopropanol) erhalten. Ausbeute 56% der Theorie.

Beispiel 8

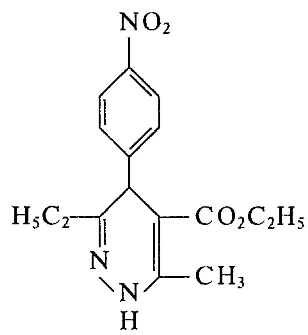

Analog Beispiel 1 wurde durch Umsetzung von 2-Acetyl-4-oxo-3-(3'-nitrophenyl)-hexancarbonsäureethylester mit Hydrazin 3-Ethyl-1,4-dihydro-6-methyl-4-(4'-nitrophenyl)-pyridazin-5-carbonsäureethylester vom Schmp. 141° C (Isopropanol) erhalten. Ausbeute 62% der Theorie.

Beispiel 9

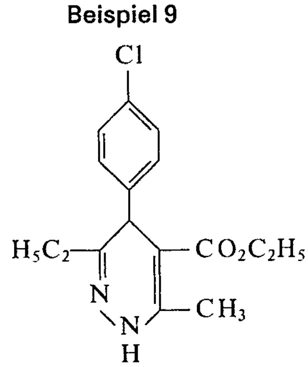

Analog Beispiel 1 wurde durch Umsetzung von 2-Acetyl-3-(4'-Morphenyl)-4-oxo-hexancarbonsäureethylester mit Hydrazin 3-Ethyl-4-(4'-chlorphenyl)-1,4-dihydro-6-methyl-pyridazin-5-carbonsäureethylester vom Schmp. 111° C (Isopropanol) erhalten. Ausbeute 39% der Theorie.

Beispiel 10

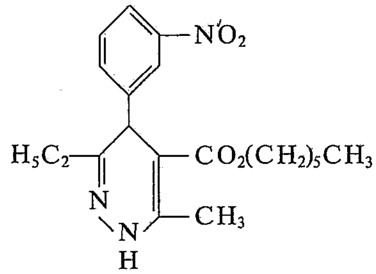

Analog Beispiel 1 wurde durch Umsetzung von 2-Acetyl-3-(3'-nitrophenyl)-4-oxo-hexancarbonsäure-n-hexylester mit Hydrazin 3-Ethyl-1,4-dihydro-6-methyl-4-(3'-nitrophenyl)-pyridazin-5-carbonsäure-n-hexylester vom Schmp. 92° C (Isopropanol) erhalten. Ausbeute 83% der Theorie.

Beispiel 11

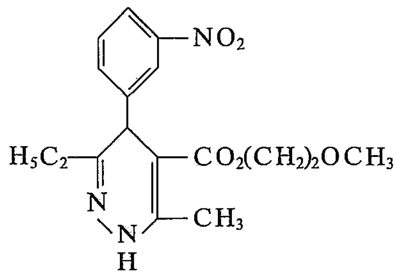

Analog Beispiel 1 wurde durch Umsetzung von 2-Acetyl-3-(3'-nitrophenyl)-4-oxo-hexancarbonsäure-($\beta$-methoxyethyl)-ester mit Hydrazin 3-Ethyl-1,4-dihydro-6-methyl-4-(3'-nitrophenyl)-pyridazin-5-carbonsäure-($\beta$-methoxyethyl)-ester vom Schmp. 141° C (Isopropanol) erhalten. Ausbeute 67% der Theorie.

Beispiel 12

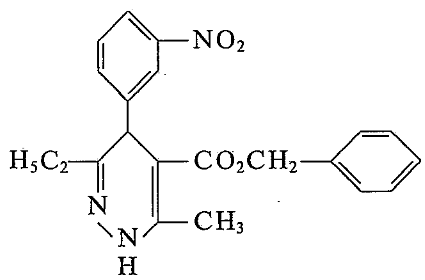

Analog Beispiel 1 wurde durch Umsetzung von 2-Acetyl-3-(3'-nitrophenyl)-4-oxo-hexancarbonsäurebenzylester mit Hydrazin 3-Ethyl-1,4-dihydro-6-methyl-4-(3'-nitrophenyl)-pyridazin-5-carbonsäurebenzylester vom Schmp. 154° C (Isopropanol) erhalten. Ausbeute 43% der Theorie.

Beispiel 13

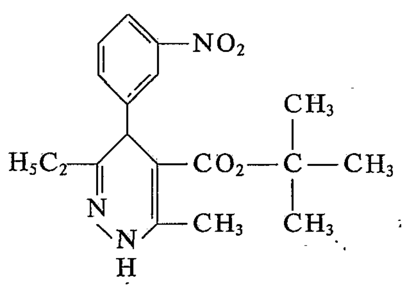

Analog Beispiel 1 wurde durch Umsetzung von 2-Acetyl-3-(3'-nitrophenyl)-4-oxo-hexancarbonsäure-t-butylester mit Hydrazin 3-Ethyl-1,4-dihydro-6-methyl-4-(3'-nitrophenyl)-pyridazin-5-carbonsäure-t-butylester vom Schmp. 122° C (Isopropanol) erhalten. Ausbeute 26% der Theorie.

Beispiel 14

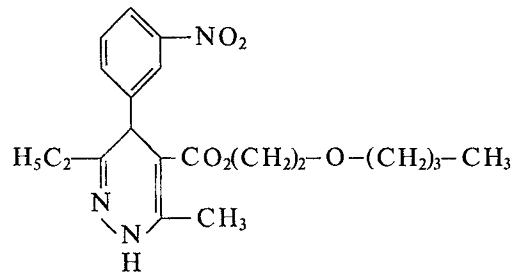

Analog Beispiel 1 wurde durch Umsetzung von 2-Acetyl-3-(3'-nitrophenyl)-4-oxo-hexancarbonsäure-($\beta$-n-butoxyethyl)-ester mit Hydrazin 3-Ethyl-1,4-dihydro-6-methyl-4-(3'-nitrophenyl)-pyridazin-5-carbonsäure-($\beta$-n-butoxyethyl)-ester vom Schmp. 122°C (Isopropanol) erhalten. Ausbeute 72% der Theorie.

Beispiel 15

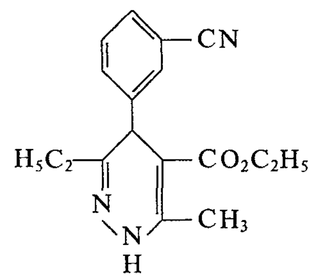

Analog Beispiel 1 wurde durch Umsetzung von 2-Acetyl-3-(3'-cyanophenyl)-4-oxo-hexancarbonsäureethylester mit Hydrazin 3-Ethyl-4-(3'-cyanophenyl)-1,4-dihydro-6-methyl-pyridazin-5-carbonsäureethylester vom Schmp. 91°C (Isopropanol) erhalten. Ausbeute 38% der Theorie.

Beispiel 16

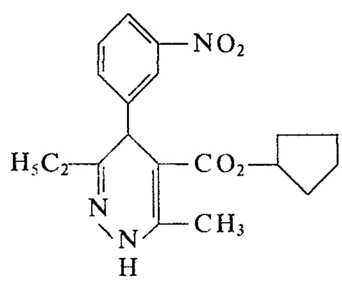

Analog Beispiel 1 wurde durch Umsetzung von 2-Acetyl-3-(3'-nitrophenyl)-4-oxo-hexancarbonsäurecyclopentylester mit Hydrazin 3-Ethyl-1,4-dihydro-6-methyl-4-(3'-nitrophenyl)-pyridazin-5-carbonsäurecyclopentylester vom Schmp. 110°C (Isopropanol) erhalten. Ausbeute 68% der Theorie.

Beispiel 17

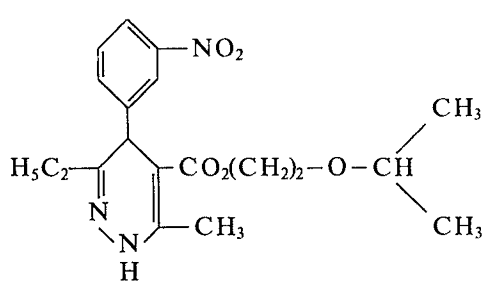

Analog Beispiel 1 wurde durch Umsetzung von 2-Acetyl-3-(3'-nitrophenyl)-4-oxo-hexancarbonsäure-($\beta$-i-propoxyethyl)-ester mit Hydrazin 3-Ethyl-1,4-dihydro-6-methyl-4-(3'-nitrophenyl)-pyridazin-5-

carbonsäure-(β-i-propoxyethyl)-ester vom Schmp. 84°C (Isopropanol) erhalten. Ausbeute 46% der Theorie.

Beispiel 18

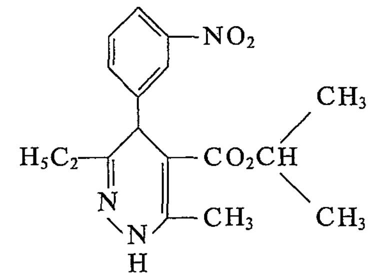

Analog Beispiel 1 wurde durch Umsetzung von 2-Acetyl-3-(3'-nitrophenyl)-4-oxo-hexancarbonsäureisopropylester mit Hydrazin 3-Ethyl-1,4-dihydro-6-methyl-4-(3'-nitrophenyl)-pyridazin-5-carbonsäureisopropylester vom Schmp. 108°C (Isopropanol) erhalten. Ausbeute 42% der Theorie.

Beispiel 19

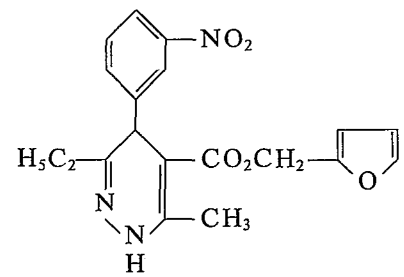

Analog Beispiel 1 wurde durch Umsetzung von 2-Acetyl-3-(3'-nitrophenyl)-4-oxo-hexancarbonsäurefurfurylester mit Hydrazin 3-Ethyl-1,4-dihydro-6-methyl-4-(3'-nitrophenyl)-pyridazin-5-carbonsäurefurfurylester vom Schmp. 152°C (Isopropanol) erhalten. Ausbeute 80% der Theorie.

Beispiel 20

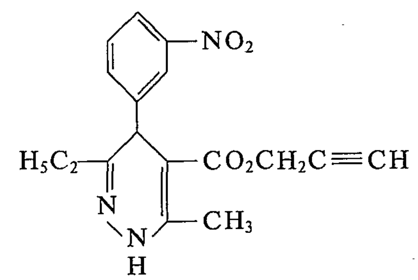

Analog Beispiel 1 wurde durch Umsetzung von 2-Acetyl-3-(3'-nitrophenyl)-4-oxo-hexancarbonsäurepropargylester mit Hydrazin 3-Ethyl-1,4-dihydro-6-methyl-4-(3'-nitrophenyl)-pyridazin-5-carbonsäurepropargylester vom Schmp. 70°C (Isopropanol) erhalten. Ausbeute 29% der Theorie.

Beispiel 21

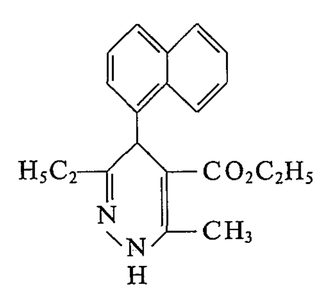

Analog Beispiel 1 wurde durch Umsetzung von 2-Acetyl-3-α-naphthyl-4-oxo-hexancarbonsäureethylester mit Hydrazin 3-Ethyl-1,4-dihydro-6-methyl-4-α-naphthyl-pyridazin-5-carbonsäureethylester vom Schmp. 122°C (Isopropanol) erhalten. Ausbeute 72% der Theorie.

Beispiel 22

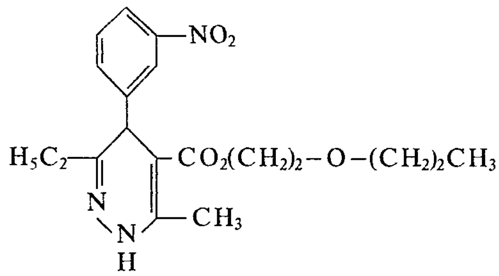

Analog Beispiel 1 wurde durch Umsetzung von 2-Acetyl-3-(3'-nitrophenyl)-4-oxo-hexancarbonsäure-(β-n-propoxyethyl)-ester mit Hydrazin 3-Ethyl-1,4-dihydro-6-methyl-4-(3'-nitrophenyl)-pyridazin-5-carbonsäure-(β-n-propoxyethyl)-ester vom Schmp. 76°C (Isopropanol) erhalten. Ausbeute 22% der Theorie.

Beispiel 23

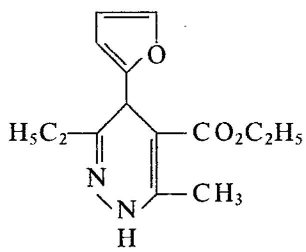

Analog Beispiel 1 wurde durch Umsetzung von 2-Acetyl-4-(furyl-(2))-4-oxo-hexancarbonsäureethylester mit Hydrazin 3-Ethyl-dihydro-4-(furyl-(2))-6-methyl-pyridazin-5-carbonsäureethylester vom Schmp. 82°C (Isopropanol) erhalten. Ausbeute 49% der Theorie.

Beispiel 24

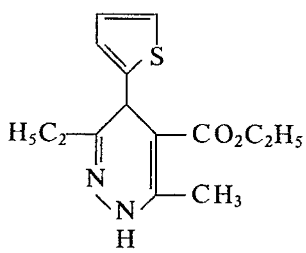

Analog Beispiel 1 wurde durch Umsetzung von 2-Acetyl-4-oxo-3-(thienyl-(2))-hexancarbonsäureethylester mit Hydrazin 3-Ethyl-1,4-dihydro-6-methyl-4-(thienyl-(2))-pyridazin-5-carbonsäureethylester vom Schmp. 127°C (Isopropanol) erhalten.

Beispiel 25

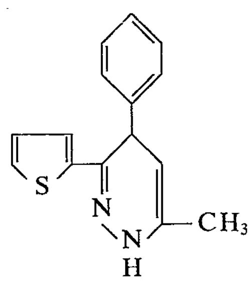

Analog Beispiel 1 wurde durch Umsetzung von 2-Phenyl-1-(thienyl)-(2))-pentandion-1,4 mit Hydrazin 1,4-Dihydro-6-methyl-4-phenyl-3-(thienyl-(2))-pyridazin vom Schmp. 150°C erhalten. Ausbeute 83% der Theorie.

Beispiel 26

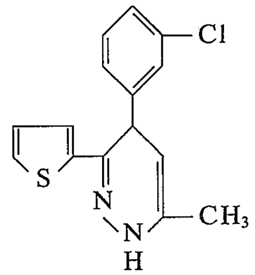

Analog Beispiel 1 wurde durch Umsetzung von 2-(3′-Chlorphenyl)-1-(thienyl-(2))-pentandion-1,4 mit Hydrazin 4-(3′-Chlorphenyl)-1,4-dihydro-6-methyl-3-(thienyl-(2))-pyridazin vom Schmp. 109° C (Isopropanol) erhalten. Ausbeute 86% der Theorie.

Beispiel 27

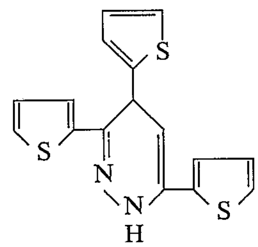

Analog Beispiel 1 wurde durch Umsetzung von 1,2,4-Tri-(thienyl-(2))-butandion-1,4 mit Hydrazin 1,4-Dihydro-3,4,6-tri-(thienyl-(2))-pyridazin vom Schmp. 168° C (Isopropanol) erhalten. Ausbeute 78% der Theorie.

Beispiel 28

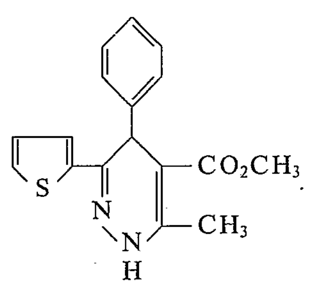

Analog Beispiel 1 wurde durch Umsetzung von 2-Acetyl-4-oxo-3-phenyl-4-(thienyl-(2))-buttersäuremethylester mit Hydrazin 1,4-Dihydro-6-methyl-4-phenyl-3-(thienyl-(2))-pyridazin-5-carbonsäuremethylester vom Schmp. 194° C (Isopropanol) erhalten. Ausbeute 38% der Theorie.

Beispiel 29

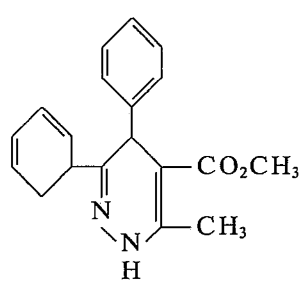

Analog Beispiel 1 wurde durch Umsetzung von 2-Acetyl-3-benzoyl-3-phenyl-propionsäuremethylester mit Hydrazin 1,4-Dihydro-3,4-diphenyl-6-methyl-pyridazin-5-carbonsäuremethylester vom Schmp. 158° C (Isopropanol) erhalten. Ausbeute 24% der Theorie.

Beispiel 30

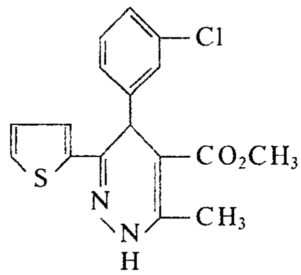

Analog Beispiel 1 wurde durch Umsetzung von 2-Acetyl-3-(3′-chlorphenyl)-4-oxo-4-(thienyl-(2))-buttersäuremethylester mit Hydrazin 4-(3′-Chlorphenyl)-1,4-dihydro-6-methyl-3-(thienyl-(2))-pyridazin-5-carbonsäuremethylester vom Schmp. 191° C (Isopropanol) erhalten. Ausbeute 18% der Theorie.

Beispiel 31

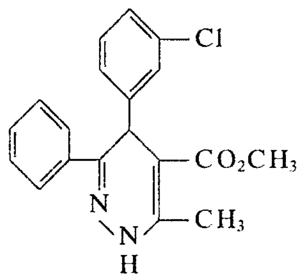

Analog Beispiel 1 wurde durch Umsetzung von 2-Acetyl-3-benzoyl-3-phenylpropionsäuremethylester mit Hydrazin 4-(3′-Chlorphenyl)-1,4-dihydro-6-methyl-3-phenyl-pyridazin-5-carbonsäuremethylester vom Schmp. 189° C (Isopropanol) erhalten. Ausbeute 15,7% der Theorie.

Beispiel 32

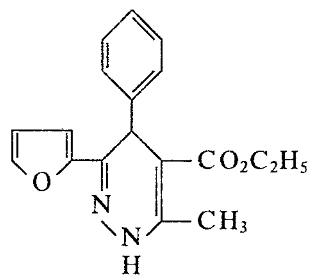

Analog Beispiel 1 wurde durch Umsetzung von 2-Acetyl-4-(furyl-(2))-4-oxo-3-phenyl-buttersäureethylester mit Hydrazin 1,4-Dihydro-3-(furyl-(2))-6-methyl-4-phenyl-pyridazin-5-carbonsäureethylester vom Schmp. 158° C (Isopropanol) erhalten. Ausbeute 48% der Theorie.

Beispiel 33

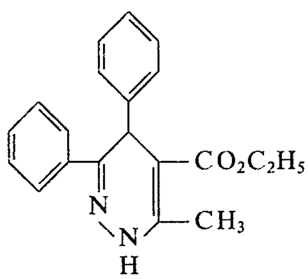

Analog Beispiel 1 wurde durch Umsetzung von 2-Acetyl-3-benzoyl-3-phenyl-propionsäureethylester mit Hydrazin 1,4-Dihydro-3,4-diphenyl-6-methyl-pyridazin-5-carbonsäureethylester vom Schmp. 130° C erhalten. Ausbeute 18% der Theorie.

Beispiel 34

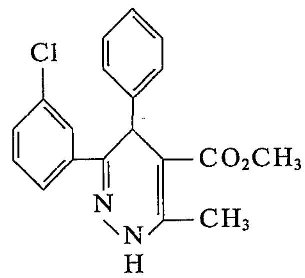

Analog Beispiel 1 wurde durch Umsetzung von 2-Acetyl-3-(3'-chlorbenzoyl)-3-phenyl-propionsäuremethylester mit Hydrazin 3-(3'-Chlorphenyl)-1,4-dihydro-6-methyl-4-phenyl-pyridazin-5-carbonsäuremethylester vom Schmp. 176° C (Isopropanol) erhalten. Ausbeute 26% der Theorie.

Beispiel 35

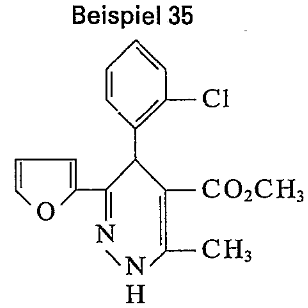

Analog Beispiel 1 wurde durch Umsetzung von 2-Acetyl-3-(2'-chlorphenyl)-4-(furyl-(2))-4-oxo-buttersäuremethylester mit Hydrazin 4-(2'-Chlorphenyl)-1,4-dihydro-3-(furyl-(2))-6-methyl-pyridazin-5-carbonsäuremethylester vom Schmp. 195° C (Isopropanol) erhalten. Ausbeute 24% der Theorie.

Beispiel 36

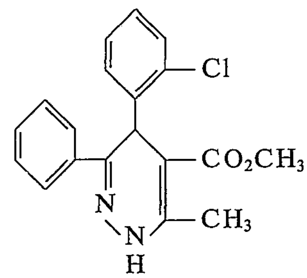

Analog Beispiel 1 wurde durch Umsetzung von 2-Acetyl-3-benzoyl-3-(2'-chlorphenyl)-propionsäuremethylester mit Hydrazin 4-(2'-Chlorphenyl)-1,4-dihydro-6-methyl-3-phenyl-pyridazin-5-carbonsäuremethylester vom Schmp. 198° C (Isopropanol) erhalten. Ausbeute 18,9% der Theorie.

Beispiel 37

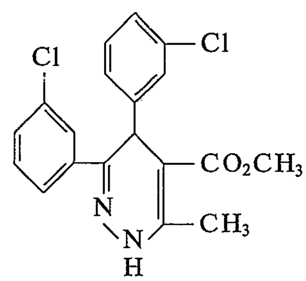

Analog Beispiel 1 wurde durch Umsetzung von 2-Acetyl-3-(3'-chlorbenzoyl)-3'-chlorphenyl)-propionsäuremethylester mit Hydrazin 3,4-Di-(3'-chlorphenyl)-1,4-dihydro-6-methyl-pyridazin-5-carbonsäuremethylester vom Schmp. 204° C (Isopropanol) erhalten. Ausbeute 12,4% der Theorie.

Beispiel 38

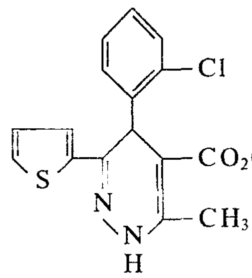

Analog Beispiel 1 wurde durch Umsetzung von 2-Acetyl-3-(2'-chlorphenyl)-4-oxo-4-(thienyl-(2))-buttersäuremethylester mit Hydrazin 4-(2'-Chlorphenyl)-1,4-dihydro-6-methyl-3-(thienyl-(2))-pyridazin-5-carbonsäure vom Schmp. 188° C (Isopropanol) erhalten. Ausbeute 18,3% der Theorie.

Beispiel 39

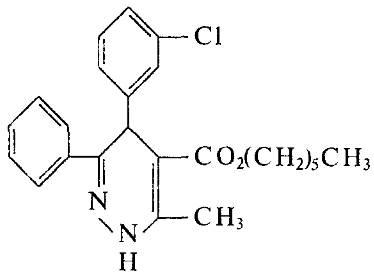

Analog Beispiel 1 wurde durch Umsetzung von 2-Acetyl-3-benzoyl-3-(3'-chlorphenyl)-propionsäure-n-hexylester mit Hydrazin 4-(3'-Chlorphenyl)-1,4-dihydro-6-methyl-3-phenyl-pyridazin-5-carbonsäure-n-hexylester vom Schmp. 103° C (Isopropanol) erhalten. Ausbeute 45,1% der Theorie.

Beispiel 40

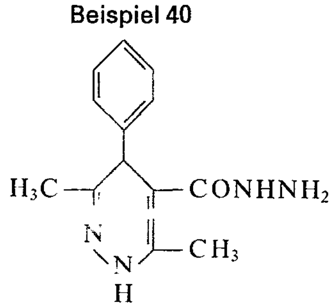

Analog Beispiel 1 wurde durch Umsetzung von 2-Acetyl-4-oxo-3-phenyl-pentancarbonsäurehydrazid mit Hydrazin 1,4-Dihydro-3,6-dimethyl-4-phenyl-pyridazin-5-carbonsäurehydrazid vom Schmp. 156° C (Ethanol) erhalten. Ausbeute 32% der Theorie.

Beispiel 41

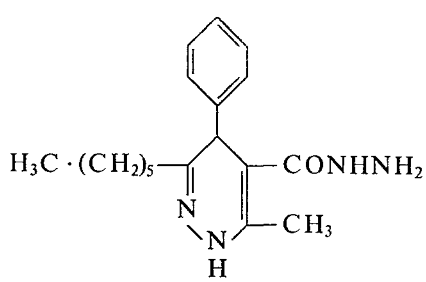

Analog Beispiel 1 wurde durch Umsetzung von 2-Acetyl-4-oxo-3-phenyl-decancarbonsäurehydrazid mit Hydrazin 1,4-Dihydro-6-methyl-3-n-pentyl-4-phenyl-pyridazin-5-carbonsäurehydrazid vom Schmp. 161° C (Ethanol) erhalten. Ausbeute 29% der Theorie.

Beispiel 42

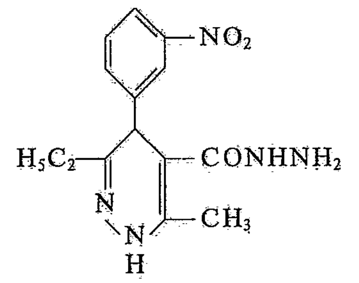

Analog Beispiel 1 wurde durch Umsetzung von 2-Acetyl-4-oxo-3-(3'-nitrophenyl)-hexancarbonsäurehydrazid mit Hydrazin 3-Ethyl-1,4-dihydro-6-methyl-4-(3'-nitrophenyl)-pyridazin-5-carbonsäurehydrazid vom Schmp. 98° C (Ethanol) erhalten. Ausbeute 28% der Theorie.

**Patentansprüche**

1. 1,4-Dihydropyridazine der allgemeinen Formel (I)

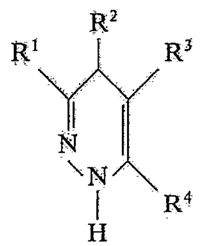

(I)

in welcher

$R^1$ für Wasserstoff, geradkettiges, verzweigtes oder cyclisches Alkyl oder Alkenyl mit bis zu 8 Kohlenstoffatomen steht, wobei die Alkylkette gegebenenfalls durch ein Sauerstoffatom unterbrochen ist oder wobei die Alkylkette substituiert ist durch Halogen, Phenoxy, Halogenphenyl, Nitrophenyl, Pyridyl, Furyl, Thienyl, Dialkyl- oder Benzylalkylamin mit je 1 bis 2 Kohlenstoffatomen im Alkylrest, oder für einen Pyridyl-, Furyl-, Thienyl-, Pyrryl- oder Phenylrest steht, wobei der Phenylrest gegebenenfalls durch 1 oder 2 gleiche oder verschiedene Substituenten aus der Gruppe Halogen, Nitro, Cyano, Trifluormethyl, Trifluormethoxy, Alkyl oder Alkoxy mit jeweils 1 bis 4 Kohlenstoffatomen substituiert ist,

$R^2$ für einen Phenylrest steht, der gegebenenfalls durch 1 oder 2 gleiche oder verschiedene Substituenten aus der Gruppe Halogen, Nitro, Cyano, Trifluormethyl, Trifluormethoxy, Alkyl, Alkenyl, Alkoxy oder Alkylmercapto mit jeweils 1 bis 4 Kohlenstoffatomen substituiert ist, oder für einen Naphthyl-, Pyridyl-, Thienyl-, Pyrryl- oder Furylrest steht,

$R^3$ für Wasserstoff oder die Gruppe $COR^5$ steht, wobei $R^5$ für einen Hydrazinorest oder einen Alkylrest mit 1 bis 4 Kohlenstoffatomen steht, oder $R^5$ und $R^4$ gemeinsam einen 5- bis 6gliedrigen Ring bilden, der gegebenenfalls noch ein Sauerstoffatom als Heteroatom enthält, oder wobei $R^5$ für die Gruppe $OR^6$, wobei $R^6$ für geradkettiges, verzweigtes oder cyclisches Alkyl, Alkenyl oder Alkinyl mit bis zu 6 Kohlenstoffatomen steht, wobei die Alkyl- und Alkenylgruppe gegebenenfalls durch ein Sauerstoffatom unterbrochen ist, oder wobei die Alkylkette gegebenenfalls substituiert ist durch Phenyl, Phenoxy, Halogenphenyl, Nitrophenyl, Pyridyl, Dialkylamin oder Benzylalkylamin mit jeweils 1 bis 2 Kohlenstoffatomen im Alkylrest, und

$R^4$ für einen Alkylrest mit 1 bis 4 Kohlenstoffatomen steht, der mit $R^5$ einen 5- bis 6gliedrigen Ring bilden kann, der gegebenenfalls als Heteroatom ein Sauerstoffatom enthält oder für einen Phenyl-, Benzyl-, Pyridyl- oder Thienylrest steht.

2. Verfahren zur Herstellung von 1,4-Dihydropyridazinen der Formel (I) gemäß Anspruch 1, dadurch gekennzeichnet, daß man 1,4-Dicarbonylverbindungen der Formel (II)

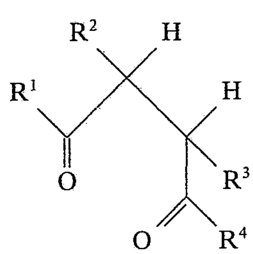

(II)

in welcher

$R^1$, $R^2$, $R^3$ und $R^4$ die oben angegebene Bedeutung haben, mit Hydrazin der Formel (III)

$$H_2N-NH_2 \qquad (III)$$

in organischen Lösungsmitteln umsetzt.

3. Arzneimittel, enthaltend mindestens ein 1,4-Dihydropyridazin gemäß Anspruch 1.

## Claims

1. 1,4-dihydropyridazines of the general formula I

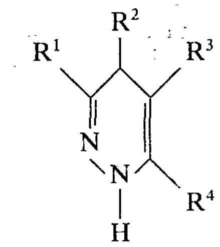

(I)

in which

$R^1$ represents hydrogen, straight-chain, branched or cyclic alkyl or alkenyl having up to 8 carbon atoms, the alkyl chain being optionally interrupted by an oxygen atom, or the alkyl chain being substituted by halogen, phenoxy, halogenophenyl, nitrophenyl, pyridyl, furyl, thienyl or dialkylamino or benzylalkylamino having 1 to 2 carbon atoms in the alkyl moiety in each case, or represents a pyridyl, furyl, thienyl, pyrryl, or phenyl radical, the phenyl radical optionally being substituted by one or two identical or different substituents from the group comprising halogen, nitro, cyano, trifluoromethyl, trifluoromethoxy, alkyl or alkoxy having in each case from 1 to 4 carbon atoms,

$R^2$ represents a phenyl radical which is optionally substituted by one or two identical or different substituents from the group comprising halogen, nitro, cyano, trifluoromethyl, trifluoromethoxy, alkyl, alkenyl, alkoxy or alkylmercapto having in each case from 1 to 4 carbon atoms, or represents a naphthyl, pyridyl, thienyl, pyrryl or furyl radical,

$R^3$ represents hydrogen or the group $COR^5$ wherein $R^5$ represents a hydrazino or an alkyl radical having from 1 to 4 carbon atoms, or $R^5$ and $R^4$ together form a 5-membered to 6-membered ring which optionally also contains an oxygen atom as a hetero-atom, or wherein $R^5$ represents the group $OR^6$, wherein $R^6$ represents straight-chain, branched or cyclic alkyl, alkenyl or alkinyl having up to 6 carbon atoms, the alkyl and alkenyl group being optionally interrupted by an oxygen atom or the alkyl chain being optionally substituted by phenyl, phenoxy, halogenophenyl, nitrophenyl, pyridyl, dialkylamino or benzylalkylamino having in each case 1 to 2 carbon atoms in the alkyl moiety, and

$R^4$ represents an alkyl radical having from 1 to 4 carbon atoms, which together with $R^5$ can form a 5-membered to 6-membered ring, which optionally contains an oxygen atom as hetero atom or represents a phenyl, benzyl, pyridyl or thienyl radical.

2. A process for the production of 1,4-dihydropyridazines of the formula (I) according to claim 1, characterised in that 1,4-dicarbonyl compounds of the formula (II)

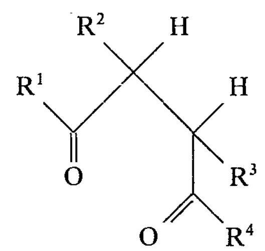

(II)

in which

$R^1$, $R^2$, $R^3$ and $R^4$ have the meaning indicated above are reacted with hydrazine of the formula (III)

$$H_2N-NH_2 \qquad (III)$$

in organic solvents.

3. Medicament containing at least one 1,4-dihydropyridazine according to claim 1.

## Revendications

1. Des 1,4-dihydropyridazines de formule générale (I):

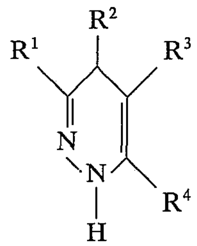

(I)

dans laquelle:

$R^1$ est de l'hydrogène, un groupe alkyle ou alcényle à chaîne droite, à chaîne ramifiée ou cyclique ayant jusqu'à 8 atomes de carbone, la chaîne alkylique étant éventuellement interrompue par un atome d'oxygène ou bien substituée par un halogène, un reste phénoxy, halogénophényle, nitrophényle, pyridyle, furyle, thiényle, dialkyl- ou benzylalkylamino ayant chacun 1 ou 2 atomes de carbone dans le reste alkyle, ou un reste pyridyle, furyle, thiényle, pyrryle ou phényle, le reste phényle portant éventuellement un ou deux substituants égaux ou différents choisis entre un halogène et des radicaux nitro, cyano, trifluorométhyle, trifluorométhoxy, alkyle ou alkoxy ayant chacun 1 à 4 atomes de carbone,

$R^2$ désigne un reste phényle qui est éventuellement substitué par un ou deux substituants égaux ou différents choisis entre un halogène et des radicaux nitro, cyano, trifluorométhyle, trifluorométhoxy, alkyle, alcényle, alkoxy ou alkylmercapto ayant chacun 1 à 4 atomes de carbone ou un reste naphtyle, pyridyle, thiényle, pyrryle ou furyle,

$R^3$ est de l'hydrogène ou le groupe $COR^5$ dans lequel $R^5$ représente un reste hydrazino ou un reste alkyle ayant 1 à 4 atomes de carbone, ou bien $R^5$ et $R^4$ forment ensemble un noyau pentagonal ou hexagonal qui comprend encore éventuellement un atome d'oxygène comme hétéro-atome, ou bien $R^5$ représente le groupe $OR^6$ dans lequel $R^6$ est un radical alkyle, alcényle ou alcynyle à chaîne droite, à chaîne ramifiée ou cyclique ayant jusqu'à 6 atomes de carbone, le groupe alkyle ou alcényle étant éventuellement interrompu par un atome d'oxygène, ou bien la chaîne alkylique étant éventuellement substituée par un reste phényle, phénoxy, halogénophényle, nitrophényle, pyridyle, dialkylamino ou benzylalkylamino ayant chacun 1 ou 2 atomes de carbone dans le reste alkyle, et

$R^4$ est un reste alkyle ayant 1 à 4 atomes de carbone, qui peut former avec $R^5$ un noyau pentagonal ou hexagonal renfermant, le cas échéant, un atome d'oxygène comme hétéro-atome, ou un reste phényle, benzyle, pyridyle ou thiényle.

2. Procédé de production de 1,4-dihydropyridazines de formule (I) suivant la revendication 1, caractérisé en ce qu'on fait réagir des composés 1,4-dicarbonyliques de formule (II):

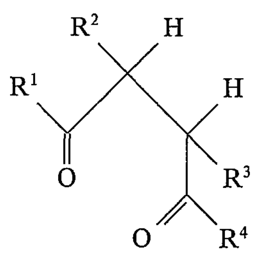

(II)

dans laquelle:

$R^1$, $R^2$, $R^3$ et $R^4$ ont la définition donnée cidessus, avec l'hydrazine de formule (III):

$$H_2N—NH_2 \quad \text{(III)}$$

dans des solvants organiques.

3. Des médicaments contenant au moins une 1,4-dihydropyridazine suivant la revendication 1.